# EUROPEAN PATENT APPLICATION

(11) **EP 4 517 771 A1**
(43) Date of publication of application: **05.03.2025**
(21) Application number: 23195096.5
(22) Date of filing: 04.09.2023
(51) Int. Cl.: G16H 40/20, G16H 40/63

(54) **METHOD FOR PROVIDING EXPERIENCE DATA**

(71) Applicant: Koninklijke Philips N.V., 5656 AG Eindhoven (NL)
(72) Inventor: BULUT, Murtaza, Eindhoven (NL); KLAASSEN, Remy, Eindhoven (NL); NAUTS, Sanne, 5656 AG Eindhoven (NL); VAN EE, Raymond, Eindhoven (NL)
(74) Representative: Philips Intellectual Property & Standards

(57) **Abstract**

A computer implemented method for providing experience data for adapting an experience in a medical environment, comprising the steps: providing an experience mode model configured to describe a relation between workflow information data and/or protocol information data and experience data for adapting an environment (S100); obtaining workflow information data and/or protocol information data (S200); generating experience data for adapting an experience based on the obtained workflow information data and/or protocol information data and the experience mode model (S300); providing the generated experience data for further processing (S400).

## Description

### FIELD OF THE INVENTION

The present invention relates to a computer implemented method for providing experience data for adapting an experience in a medical environment, to a data processing apparatus, to a computer program and to a computer readable medium.

### BACKGROUND OF THE INVENTION

Ambient experience systems are used to provide a pleasant atmosphere in medical environments, such as a CT room or a corresponding waiting room. Ambient experience systems may allow to adapt one or more devices, such as a light or a display in the medical environment. These systems directly or indirectly influence patients and staff and therefore help to improve healthcare operations. There exist different targets and/or strategies how to adapt such a medical environment. Currently, these system are operated manually by staff members to create an experience in a medical environment.

### SUMMARY OF THE INVENTION

There may, therefore, be a need for a method for providing experience data for automatically adapting an experience in a medical environment. The object of the present invention is solved by the subject matter of the independent claims, wherein further embodiments are incorporated in the dependent claims.

According to a first aspect, there is provided a computer implemented method for providing experience data for adapting an experience in a medical environment, comprising the steps:
providing an experience mode model configured to describe a relation between workflow information data and/or protocol information data and experience data for adapting an environment;
obtaining workflow information data and/or protocol information data;
generating experience data for adapting an experience based on the obtained workflow information data and/or protocol information data and the experience mode model;
providing the generated experience data for further processing.

The invention is based on the finding that so-called ambient experience systems are used in combination with different operations and healthcare environments. These ambient experience systems will provide the possibility to create a pleasant atmosphere to directly and indirectly influence patients in order to improve healthcare operations. The ambient experience system allows a user (e.g., staff or physician) to adapt manually one or more devices in a medical environment that are connected to the ambient experience system, such as lightning in a medical environment, a screen, and/or a sound system. The systems do not consider any workflow information and are run manually. For example, in a typical implementation a playlist of difference experiences is pre-created, playing of the playlist is manually initiated by the staff and playing of the playlist is manually stopped by the staff.

The invention proposes to generate such an experience based on workflow information data and/or protocol information data automatically. This may allow to improve patient experience or staff experience during a procedure as it matches more the requirements in a current workflow step. A medical procedure comprises a plurality of workflow steps and during these workflow steps different actions may be carried out. These different actions may require different experiences for staff and or patients. The targeted adaption of such an experience to a specific action may improve the performance of a corresponding action performed in such a workflow step. For example, if a patient is relaxed due to smoothing music, the medical procedures may be performed faster and more accurately. The medical staff may benefit as well as specific information needed (notification, data, checklist) in a workflow step may be presented on a screen to them right now when it is needed. The method may allow to address both needs of patient and staff simultaneously.

In other words, the ambient experience systems are currently run manually and therefore require an effort of the staff members to operate them. Generally, this may result in an additional working time, a suboptimal operation of the ambient experience system and a suboptimal creation of an experience during different workflow steps in a medical procedure. The invention overcomes these drawbacks. In a non-limiting and explanatory embodiment, the method obtains automatically workflow information such as a start of a next workflow step, current positions of staff members and patients. The method processes these information and adapts automatically based on the processed information devices (e.g., positions of screens, appropriate content on these screens, and light conditions of lights). This may lead to an optimal experience for staff members, physicians, and patients. This may lead to an increase of the procedure efficiency as the method provides optimal working conditions due to the optimized experience in the medical environment.

The term "experience data", as used herein, is to be understood broadly and may relate to control data for adapting one or more devices in a medical environment in order to adapt an experience in the medical environment.

The experience data may be used to adjust a content and/or a position of a screen in a medical environment. For example, this may entail positioning a display with respect to a MR equipment and patient head, or control room location can be arranged in such a way that multi-view displays can be generated with a high quality. The display can physically move up-down, left-right to enable the best viewing angle for the patient and the staff. The position (or head position) of patient and staff can be tracked, and the display position can be adjusted accordingly, for example after the scan has begun, allowing minimal interference and best image quality for the different types of content consumed by the staff and the patient. Content may be selected and tuned based on the user height and possible rendering possibilities of dual experiences to enable a high quality.

The experience data may entail control such that the content may follow a patient or staff for an immersive experience. Based on obtained workflow information and/or workflow progress information each by tracking a viewing angle of a patient, a content may be accordingly adapted to generate an immersive experience (e.g., 3D experience). This may allow to provide an uninterrupted 3-D experience for different positions of the patient from when patient enters the room (standing & walking), patient sitting on the table, to patient laying on the table. Digital light processing technology can be used for the implementation.

The experience data may entail personalized content based on workflow information data and/or workflow progress data. The workflow information data may be obtained by tracking. The workflow information may be obtained from electronic medical records (EMR) or any other hospital information system. The workflow progress data may be obtained by tracking. The workflow information data and/or workflow progress data may comprise a staff role or staff identification. Based on the role or the identification, previous viewed content may be determined. This experience data may entail such previous viewed content. This may entail an authorization of content (e.g., one staff member may be allowed to view the content, another staff member may be not allowed). The experience data may also comprise preference of content associated with a staff role or staff identification. The workflow information and/or workflow progress data may comprise a patient identification. The workflow information data and/or workflow progress data may comprise a preference associated to a specific patient. For example, the preference may be an additional clinical measure for the specific patient, such as a blood collection. For example, the preference may be a specific light adaption for a patient suffering from claustrophobia. The workflow information data may reveal for a specific patient that a certain process step may take more time in comparison to a standard patient. The workflow information data and/or workflow progress data may comprise personalized content for the specific patient (e.g. records of the course of the disease, notes for this specific patient). The experience data may take into account such patient specific aspects.

The experience data may further comprise content data, such as for example procedure information, images or videos. The content data may be time specific. The content data may be user specific. The content data may stem from libraries. The content data may stem from real time imaging during a procedure. The content data may stem from data bases (e.g., data of a previous procedure (e.g., protocols)).

The term "experience", as used herein, is to be understood broadly and may relate to an atmosphere in a medical environment. The atmosphere may be described by positions of one or more devices (e.g., screen oriented to a patient or to a staff), the state of one or more devices (e.g., screen on or off, light on or off, music off or quiet or loud), the content transmitted by one or more devices (e.g., images, videos, music).

The term "medical environment", as used herein, is to be understood broadly and relates to any medical facility used for procedure. Preferably the term medical environment relates to an imaging environment, such as a CT room, a digital x-ray radio grammetry room, an image guided therapy (IGT) room, a MRI room. The medical environment may further comprise a corresponding waiting room and/or a corresponding recovery room.

The term "experience mode model", as used herein, is to be understood broadly and may relate to any logic to describe a relation between workflow information data and/or protocol information data, and experience data for adapting an environment. The experience mode model may be a lookup table, an algebraic model, a machine learning model, a semantic model. The experience mode model may consider workflow information data for determining a workflow step in a procedure. The experience mode model may determine based on the on the obtained workflow information a start of a workflow step. The experience mode model may estimate based on workflow information data a duration of a workflow step. The experience mode model may determine based on workflow information data and an estimation of a duration of a workflow step an end of a workflow step. The experience model may receive tracked workflow information data during a procedure and may use the tracked workflow information data for determining a start, a duration, an intermediate step/stage, and an end of a workflow step. The experience mode model may use these intermediate results for determining the experience data for adapting an environment.

The term "workflow information data", as used herein, is to be understood broadly and may relate to any information configured to describe a workflow. Preferably the workflow information may relate to a workflow step of a procedure and/or an intermediate step/stage of a workflow step. The workflow step or workflow steps may be predetermined by a hospital for a medical procedure. The workflow information data may relate to a start time of a workflow step, to a duration of a workflow step, to an end time of a workflow step. The workflow information data may relate to a patient. The workflow information data may relate to a staff (e.g., nurse, physician). Preferably, the workflow information data relate to workflow steps of a procedure. Workflow steps may be for example: patient waiting in the waiting area, patient enters the procedure area, patient is prepared for the procedure, waiting for the physician, performance of procedure, patient recovers from procedure, exits the procedure area, cleaning of procedure area, patient recovering in the recovery room. The workflow information data may be obtained by tracking staff activity, patient activity, staff state, patient state, and/or usage of a device or a plurality of devices. The workflow information data may be obtained by a query of a data base, wherein the workflow and corresponding workflow steps are stored. The workflow information data may be obtained from records and/or data generated during a procedure. For example, the workflow information data may be generated by a human (e.g. clinician and/or patient). For example, the workflow information data may be generated by a sensor (e.g. sensor in a device or sensor in a medical environment). For example, the workflow information data may be generated by a device itself (e.g. log file, energy usage and/or computational resource).

The term "protocol information data", as used herein, is to be understood broadly and may relate to a guideline how to treat a patient during a procedure. The protocol information data may comprise one or more of the following: a rule, a step, an action. The rule, the step, the action may need to be executed during a procedure or when taking care of the patient. One or more rules may need to be checked during or before a step. For example, a step may be taking blood samples for testing. For example, an action may be inserting a needle into the patient. For example, a rule may be a check of exclusions criteria such as a minimum age for blood donation. The rule (e.g., a check of the rule), the step, and/or the action may be performed in an ordered manner. E.g., an execution of a current step may depend on an execution of a previous step and a patient's reaction to the previous step. The protocol information data may be personalized to a patient. The protocol information data may be stored on paper. Preferably, the protocol information data may be stored electronically. The protocol information data may be stored in a data base. For example, the protocol information data may be stored as pdf and/or in a guidance software. The described method may obtain the protocol information data by an interface to a data base and a corresponding query. An execution of the protocol information data, in particular a rule, a step, an action may be tracked (e.g., protocol guidance solution).

The term "further processing", as used herein, is to be understood broadly and may relate to using the generated experience data for controlling one or more devices. For instance, the generated experience data is used to control the movement of a screen in a MRT room in order to set up the position.

Optionally, the workflow information data may comprise an alarm signal or a probability that an alarm is being triggered.

In an embodiment, the experience mode model may further be configured to describe a relation between the workflow information data and/or the protocol information data and selection modes for an experience in the medical environment; and the method may further comprises the steps:
determining a selection mode of a plurality of selection modes based on the workflow information data and/or the protocol information data and the experience mode model; and
generating experience data for adapting an experience based on the determined selection mode.

The term "selection mode", as used herein, is to be understood broadly and may relate to a preselection of parameters of one or more devices to be adapted for setting up an experience that addresses specific actions performed by a patient and/or staff. The selection may additionally address needs of a patient and/or staff. There may be a plurality of selection modes. For instance, a patient staff communication mode or a patient data visualization mode. The patient data visualization may for instance require a specific position of a screen, a specific content on the screen, and a specific light of a light in the IGT room. The generating of the experience data based on the determined selection mode may be based on a look up table, an algebraic model, and/or a machine learning model. E.g., a specific selection mode may correspond to a specific selection of parameters and parameter values used to generate the experience data.

The experience mode model may comprise a look-up table used to determine the selection mode. For example, the look up table may comprise a mode selection rule and selection modes associated with workflow information (e.g., workflow steps). For example, the look up table may comprise a list of elements of one or more workflow steps. Based on a comparison of these elements with the workflow information and/or protocol information, a selection mode may be determined.

The experience mode model may comprise an algebraic model used to determine the selection mode. For example, the algebraic model may comprise a mathematical formula, wherein an input of the formula comprises workflow information and/or protocol information and the output of the formula comprises a selection mode. For example, the input may comprise device related parameters. For example, the device related parameters may comprise a position and/or a movement of a medical equipment. For example, the medical equipment may be a screen or a C-arm. For example, the input may comprise staff related parameters. For example, staff related parameters may comprise a position and/or a movement of staff (e.g., nurse, physician).

The experience mode model may comprise a machine learning model. The machine learning model may be trained with workflow information data and/or protocol information data as input and a corresponding timeline of a workflow, in particular of a procedure in a workflow as output (e.g., the time until the current procedure is completed, and the time until the whole workflow steps are completed).

In an embodiment, the plurality of selection modes may comprise a shared mode for a joint experience for a staff and a patient and/or an individual mode for individual experiences for a staff and a patient.

The term "joint mode", as used herein, may relate to a common experience in a medical environment for the patient and the staff. In other words, the joint mode comprises no distinction between patient or staff needs. This may for example entail that on a screen only one common content is presented. E.g., this may entail that only experience data is generated that addresses only the needs of a patient, only the needs of staff, or both the needs of a patient and staff. This may be advantageous as, for example, a content on a screen, is presented to both patient and staff without particular personalization in how it should be perceived by a specific individual (e.g. patient or staff). The term "individual mode", as used herein, may relate to a specific experience for the staff and to another specific experience for the patient. The individual mode may allow to provide different experiences to different patients or staff members at the same time. In other words, a specific (personal) experience can be generated for the patient and the staff, at different times, but also at the same time. The individual mode may comprise a distinction between patient and staff needs. This may for example entail that on a screen individual content for the staff and individual content for the patient are presented. The individual mode may also be used for different patients. For example, one patient may have an appointment for an MRI and another patient walking just behind for a CT; then for each patient a corridor screen can present specific routing information. For example, when multiple patients are present in a waiting room, one patient's protocol may describe the need to drink fluids during the waiting time before the exam, another patient just may have to wait before being called in. The experience can be joint for the waiting part of the time, for example when there may be more than 20 minutes before each patient's exam and can aim to relax both patients simultaneously; but can become separate for the patient reminding her/him to drink the fluids. The experience can also become separate when a specific patient is being called in to a certain exam room and guiding the patient to that specific room, whilst the other patients are still in in waiting mode. This may increase the performance of a medical procedure.

In an embodiment, the plurality of selection modes may comprise one or more of the following: patient staff communication mode, inter staff communication mode, patient data collection mode, procedure preparation mode, patient data visualization mode and patient data analysis mode.

Optionally, the plurality of selection modes may comprise one or more of the following: patient preparation mode, patient discharge mode, patient going-to sleep mode, patient-wake-up mode (from anesthesia) and cleaning mode.

The term "patient staff communication mode", as used herein, may relate to a mode for supporting a communication between patient and staff. For example, when the communication is for relaxing the patient, a joint mode can be used. For example, when the communication is for providing information/instructions to the patient, a separate mode can be used where the views for staff may display the information that may be needed to be communicated, and the views for the patient may provide summaries (bullet points) of the communicated information. Such a solution may help staff not to forget important details and may help patient to remember what the patient is told. The patient staff communication mode may entail a speech recognition. The patient staff communication mode may comprise displaying at least parts of patient responses on screens to staff. The patient staff communication mode may comprise displaying summaries of staff explanations to patient.

The term "inter staff communication mode", as used herein, may relate to a mode for supporting an inter-clinician communication. For example, the lighting settings can be adapted so that the monitoring parameters are easier to visualize. For example, views for staff can include information that the staff should know or pay attention to related to this particular workflow step while views from physicians may ensure that images are displayed with the sharpest intensity and greater size. The inter staff communication mode may consider whether a staff member is in the medical environment where the experience is generated or whether the staff member is outside this medical environment (e.g., remote call via video conference system or telehealth system). In case of a staff member that communicates from remote with a staff member in the medical environment, the inter staff communication mode may present information to the staff member in the medical environment considering for example his/her position in the medical environment.

The term "patient data collection mode", as used herein, may relate to a mode configured to influence the patient and to monitor his reaction. For example, the patient data collection mode may trigger eye movements of the patient by shifting a light from one side to another. For example, the patient data collection mode may monitor by a camera the eye movement of the patient. This may for example allow to determine an alertness of the patient. In another example, the patient data collection mode may trigger events (e.g., sounds, light) to evaluate a sleepiness level of the patient. The patient data collection mode may help to clinicians to perform data collection in the most effective and accurate manner. For example, by relaxing the patient, the movements can be minimized and this can speed up the data collection set up, collection itself, as well improve the quality. The data collection mode may create an environment that comprises the right assistance and conditions for data collection.

The term "procedure preparation mode", as used herein, may relate to a mode for assisting two or more clinicians that are working simultaneously on different tasks. The procedure preparation mode may provide personalized assistance. The personalized assistance may comprise checklists that each need to be followed, sound conditions, light conditions that enable the clinicians to do their work more efficiently.

The term "patient data visualization mode", as used herein, may relate to a mode for visualizing patient data in a manner such that clinicians can receive efficiently the information. For instance, lighting in the medical environment can be adjusted so that the images/videos are better visible, with greater contrast and sharpness. Optionally, a sound landscape in the medical environment can be adjusted so that the alarms are better audible by clinicians and non-audible by the patient. Optionally, colors on a screen can be adjusted so that they do not interfere with alarm colors on a patient monitor (for instance, adjusting the lighting so that yellow, red alarm are easily noticeable).

The term "patient data analysis mode", as used herein, may relate to a mode for studying patient data. The patient data analysis mode may be used for clinicians. The patient data analysis mode may comprise experience data such that a patient data analysis is simplified or improved. For example, patient data analysis may comprise comparing two previous images, or signals, to search and retrieve previous data, and to run an algorithm. The patient data analysis mode may comprise experience data configured to adapt an environment to help these activities to be performed faster and better.

In an embodiment, the method may further comprise obtaining workflow progress data; using the obtained workflow progress data for determining the selection mode; and/or using the obtained workflow progress data for generating the experience data.

The term "workflow progress data", as used herein, is to be understood broadly and may relate to any workflow data configured to describe a course of a workflow process. The workflow progress data may comprise a start of a workflow process step, and an end of a flow process step, a duration of a workflow process step, an assignment of a specific workflow step. The workflow progress data may comprise intermediate stages of a workflow step. The workflow progress data may be real time data. The workflow progress data may be obtained by a tracking unit (e.g., a CPU, a vision system or any other programmable logic) configured to automatically track which workflow step is currently applicable. For example, the workflow progress data may be obtained by tracking triggers or times. For example, after the patient is asked to enter the procedure area, the start and end time of the following steps may be approximated based on prior information or based on specific rules. For example, the activities of staff can be tracked to determine the current workflow step, and this information can be combined with prior data (or rules) to estimate the end time of the current step, start time of following steps and their durations. For example, for each of these phases within the workflow a flag can be sent to a computer program. One flag can be sent when the patient is in the waiting room, another flag when the patient departs from the waiting room, and yet another flag when the patient arrives in a next room. For example, after obtaining the workflow progress data (e.g., end of workflow step 1), the initiation of workflow step 2 may be determined and a corresponding selection mode may be determined. Based on the workflow progress data (i.e. initiation of progress step 2) and the determined selection mode experience data may be generated (e.g., in the beginning of process step 2 calming music may be played).

Optionally, the workflow progress data may be obtained by tracking one or more device settings and or one or more usages of devices. Preferably, the tracking may be directed to starts of a workflow step and/or a progress of a workflow step. An end time of a workflow step may be estimated based on predefined results and/or prior information that may take into account a current progress level in the workflow or workflow step. Typically, a workflow step may be associated with a usage of specific devices. The information of these devices (e.g., positioning, setting, power level) can advantageously be used to determine the workflow step. For example, when a device X is being used a flag may be raised to a computer program (i.e. described method) and a period of time may be estimated (based upon normative data) for a present workflow procedure. When device X stops its activity, another flag may be raised to the computer program (i.e. described method). And a period of time of actual use may be recorded and stored in a log file. Upon a start of using device Y another flag may be raised and sent to the computer program (i.e. described method).

The workflow progress data may comprise different elements/features in the medical environment. The workflow progress data may comprise one or more of the following: number of people in the room, people entering/exiting the room, people moving in the room, trajectory of movements, expertise of the different people in the room, familiarity of different people with the patient records, familiarity of the people with the procedure, number and type of devices with screens from which clinicians will need to read information, specific workflow actions such as inserting a catheter, giving anesthesia. This information may be tracked in real time. This information may be advantageously used to generate experience data. This may increase an efficiency of the method.

In an embodiment, obtaining workflow progress data may comprise a tracking. The tracking may comprise tracking one or more device settings and/or one or more usages of devices. The tracking may comprise tracking an activity of staff and/or an activity of a patient. The tracking may be carried by one or more sensors. The tracking may be carried out in real time. The tracking may comprise analyzing control data of one more devices in the medical environment. The tracking may allow to obtain current workflow information data. The current workflow information data can be used to determine workflow progress data. This may advantageously increase the quality of generating experience data.

In an embodiment, the method may further comprise: obtaining staff activity data; and using the obtained staff activity data for determining the selection mode; and/or obtaining patient state data; and using the obtained patient state data for determining the selection mode; and/or obtaining staff state data; and using the steady-state data for determining the selection mode; and/or obtaining patient activities data; and using the patient activity data for determining the selection mode.

The term "staff activity data", as used herein, is to be understood broadly and may relate to any activity carried out by staff. Staff activity data may be tracked by a use of a device by the staff, a spoken word by the staff, a presence of the staff and/or a position of the staff. Staff activity data may be obtained by tracking output generated by staff. The output may comprise a sound, a facial expression, a movement, a written input, and/or a gesture. The output may comprise interaction data (e.g. with patient a monitoring device). The staff activity data may be obtained by tracking the staff activities. Tracking the staff activities may be performed by using at least one sensor. The sensor may be one of the following: optical sensor, auditory sensor, motion sensor, touch sensor. The activities may be tracked by text processing (e.g., using natural language processing - NLP) analyzing the transcribed speech text or text entries (clinical notes, entries make in electronic medical records (EMR)) generated by staff. For example, by detecting that the staff is faced to the patient and their lips are moving, a communication-with-patient activity (event) can be detected.

The term "patient state data", as used herein, is to be understood broadly may relate to any condition configured to describe a current situation of a patient. The patient state data may relate to any state that may be important to distinguish for a particular procedure and workflow execution. The patient state data may comprise one or more of the following states: distressed, relaxed, attentive, apathetic, awake, half-awake, sleeping, confused.

The patient state data may be obtained by using one or more sensors. The sensor may comprise an optical sensor and/or audio sensor. The sensor may be located in the medical environment. The sensor may track one or more facial features of the patient. The sensor may track one or more speech features. The method may use sensor data to estimate the patient state data. The method may use a look up table for estimating the patient state data based on the obtained sensor data. Optionally in addition, monitoring equipment that is connected to a patient may be used to determine patient state data. For example, vital signs such as heart rate, heart rate variability, respiration rate, and respiration depth can be used to estimate patient state data. For example, when a patient is connected to a patient monitor, all signals and information collected by the patient monitor can be used as patient state data.

The term "staff state data", as used herein, is to be understood broadly and may relate to any condition configured to describe a current situation of staff. The staff state data may comprise one or more of the following states: focused on preparation for procedure, in need of relaxation before procedure, focused on work, tired, only partially focused on patient, staff not with patient, distracted by patient. The staff state data may be obtained by one or more sensors. The sensor may be one of the following: optical sensor, auditory sensor, motion sensor, touch sensor. The activities may be tracked by text processing (e.g., using natural language processing - NLP) analyzing the transcribed speech text or text entries (clinical notes, entries make in electronic medical records (EMR)) generated by staff. The collected sensor data may be transcribed as text. The transcribed text may be analyzed. The collected sensor data may relate to an activity and/or a state of the staff.

The term "patient activity data", as used herein, is to be understood broadly and may relate to any activity carried out by patient. The patient activity data may comprises one or more of the following states: patient moving, type of patient moving (e.g., walking, shaking), patient communicating, patient following instructions. The patient activity data may be obtained in a similar manner as staff activity data.

In sum, this may be advantageous in terms of selecting the right selection mode and therefore generating optimized experience data. This may allow to monitor or estimate patient and/or staff actions, physiological signals, psychological conditions in real-time.

In an embodiment, the method may further comprise the step: obtaining prior data, wherein the prior data may comprise one or more of the following: patient monitoring data, EMR data, comparative case data with similar workflows and/or protocols including used device data, staff profile data. And this embodiment may further comprise using the obtained prior data for determining the selection mode. The prior data may be obtained via an interface of one or more of the following: patient monitoring system, data base, EMR data base, hospital data base.

The patient monitoring data may relate to previous patient monitoring data from the same patient from previous procedures. Prior information may relate to workflow, staff activities, and patient affective states. The prior information can be in the form of data (sensors, user interface, procedure, etc.) collected by the hospital. It can also be in the form of observations and learnings extracted from clinician notes, technical notes, publications, patient interviews, and so on. For example, there may be certain stages during a workflow where the patient is the most stressed and the staff is the busiest. The data previously collected by the hospital can be analyzed with respect to similar patients and similar procedures to determine events and likelihood of these that need the attention.

This may be advantageous in terms of selecting the right selection mode and therefore generating optimized experience data.

In an embodiment, the obtained workflow information data and/or the obtained staff activity data and/or the obtained patient state data and/or the obtained prior data and/or the obtained staff state data and/or the obtained patient activity data may be used for generating the experience data.

In other words, the received data is not used only to select the right selection mode, but also to generate the experience data within the selected selection mode. This may advantageously improve the experience in the medical environment.

In an embodiment, the method may further comprise: controlling one or more devices in the medical environment based on the experience data for generating a desired environment, wherein controlling comprises one or more of the following: positioning of the device, adapting the device (e.g., adapting processing settings, movement settings), selecting and transmitting of content, adapting a user interface. For example, one or more screens may be automatically positioned such that the staff and/or the patient have an optimized view on them. For example, one or more lights may be tuned, switched on or off such that a lightning may optimized for the staff and/or the patient. For example, in a certain stage of a workflow step corresponding content may be displayed on one or more screens when it is needed. This may also entail different contents on one screen. This may optimize an efficiency of a procedure.

In an embodiment, the controlling of the one or more devices may be carried out differently for two or more patients and/or staff in the medical environment. In other words, the method takes into account the different subjects in the medical environment and optimizes for each of them the medical environment. This may comprise a weighting of different objectives. E.g., based on workflow information data and/or protocol information data weights for experience data and/or experience data of selection modes may be calculated. The weights may consider a presence or degree of one or more features of workflow information data and/or protocol information data. E.g., these weights may then be processed to determine a selection mode considering two different patients (e.g. a shared mode). This may advantageously increase an efficiency of a procedure.

In an embodiment, the medical environment may be one of the following: a CT system, an IGT system, a DXR system, an MRI system. The medical environment may comprise a waiting room, a changing room, a recovery room for each of the above mentioned systems.

In an embodiment, the selection mode model may be based on one or more of the following: a look up table, a weight function, a machine learning algorithm.

A further aspect of the present disclosure relates to a data processing apparatus comprising means for carrying out the method as described above.
The data processing apparatus may be configured to perform or induce performing of the steps of the method described above. Moreover, it may be configured to operate the devices, means, units described above. The data processing apparatus may comprise one or interfaces to exchange data and/or commands with the described devices, means, and units. The data processing apparatus can be configured to operate automatically and/or to execute the orders of a user. A computer program may be loaded into a working memory of a data processor. The data processor may thus be equipped to carry out the method according to one of the preceding embodiments. This exemplary embodiment of the invention covers both, a computer program that right from the beginning uses the invention and computer program that by means of an update turns an existing program into a program that uses invention. Further, on, the computer program might be able to provide all necessary steps to fulfil the procedure of an exemplary embodiment of the method as described above. According to a further exemplary embodiment of the present invention, a computer readable medium, such as a CD-ROM, USB stick or the like, is presented wherein the computer readable medium has a computer program stored on it which computer program is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems. However, the computer program may also be presented over a network like the World Wide Web and can be downloaded into the working memory of a data processor from such a network. According to a further exemplary embodiment of the present invention, a medium for making a computer program available for downloading is provided, which computer program is arranged to perform a method according to one of the previously described embodiments of the invention.

A further aspect of the present disclosure relates to a computer program comprising instructions which, when the program is executed by a computer, cause the computer to carry out the method as described above.

A further aspect of the present disclosure relates to a computer-readable medium comprising instructions which, when executed by a computer, cause the computer to carry out the method as described above.

It is noted that the above embodiments may be combined with each other irrespective of the aspect involved. Accordingly, the method may be combined with structural features of the system of the other aspects and, likewise, the system may be combined with features described above with regard to the method.

These and other aspects of the present invention will become apparent from and elucidated with reference to the embodiments described hereinafter.

### BRIEF DESCRIPTION OF THE DRAWINGS

Exemplary embodiments of the invention will be described in the following drawings.
Fig. 1 shows a schematic flow diagram of a method according to an exemplary embodiment of the present disclosure;
Fig. 2 shows a schematic diagram of a method according to another exemplary embodiment of the present disclosure;
Fig. 3 shows a schematic illustration of an individual mode according to an exemplary embodiment of the present disclosure; and
Fig. 4 shows a further schematic illustration of an individual mode according to an exemplary embodiment of the present disclosure.

### DETAILED DESCRIPTION OF EMBODIMENTS

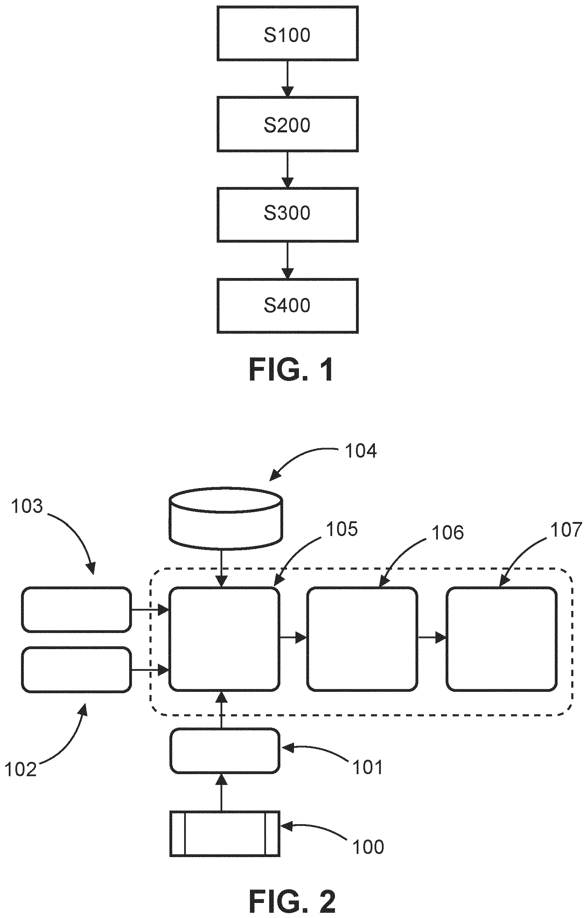

Fig. 1 shows a schematic diagram of the method according to an exemplary embodiment of the present disclosure. The computer implemented method is used for providing experience data for adapting an experience in a medical environment. The method comprises the following steps. The method may be executed by a data processing apparatus. The data processing apparatus may comprise a CPU, a memory, one or more interfaces for exchanging data with data bases, sensors, and/or devices such as displays, lights, sound systems, or projectors. The data processing apparatus may be a computer from an ambient experience system. The exchanged data may comprise sensing data, queried data from data bases, and/or control data (i.e. experience data) for adapting one or more devices in a medical environment.

Step S 100 comprises providing an experience mode model configured to describe a relation between workflow information data and/or protocol information data and experience data for adapting an experience in a medical environment. The experience mode model may be implemented as an algorithm. The algorithm may be a machine learning algorithm, a mathematical algorithm or a lookup table.

Step S 200 comprises obtaining workflow information data and/or protocol information data. The workflow information data may be obtained by a query of a data base. The protocol information data may be obtained by a query of a data base. Optionally, the workflow information data may be obtained by tracking staff activity, patient activity, staff state, patient state, and/or usage of a device or a plurality of devices. The tracking may comprise a use of one or more sensors implemented in the medical environment. The sensor may be one of the following: optical sensor, audio sensor, speech sensor. The tracking may comprise text processing (e.g., using natural language processing - NLP) analyzing the transcribed speech text or text entries (clinical notes, entries make in electronic medical records (EMR)) generated by the staff. The workflow information data may be obtained from records and/or data generated during a procedure. For example, the workflow information data may be generated by a human (e.g. clinician and/or patient). For example, the workflow information data may be generated by a sensor (e.g. sensor in a device or sensor in a medical environment). For example, the workflow information data may be generated by a device itself (e.g. log file, energy usage and/or computational resource).

Step S 300 comprises generating experience data for adapting an experience in the medical environment based on the obtained workflow information data and/or protocol information data and the experience mode model. The method may use the experience mode model to determine based on the obtained workflow information a start of a workflow step and to estimate based on workflow information data a duration of a workflow step. The method may determine based on workflow information data and an estimation of a duration of a workflow step an end of a workflow step. The method may use these intermediate results for generating the experience data for adapting an environment. For example, the generating may comprise selecting based on the intermediate results one or more experience data from a corresponding look up table.

Step S 400 comprises providing the generated experience data for further processing. In the present example, this may comprise that the respective content is transmitted to a projection unit and corresponding control data (i.e. commands) are transmitted to the projection unit for or projecting the respective content on different positions.

Optionally, the experience mode model may be configured to describe a relation between workflow information data and/or protocol information data and selection modes for an experience in the medical environment. The method may further comprise the step:
determining a selection mode of a plurality of selection modes based on the workflow information data and/or the protocol information data and the experience mode model. The workflow information data and/or protocol information data may be described as input vector. These input vectors may then be weighted by a weight function. The weighted input vectors may then be used to select a selection mode. For example, patient state data may comprise two events, distressed and other. Staff activity data may comprise two events, communicating with the patient and other. For example, in case the patient state data "distressed" is weighted higher than the staff activity data "communicating with the patient", an individual mode is selected otherwise a shared mode is selected. Alternatively, the selection mode may be a mode dedicated to improving certain action, patient staff communication mode, inter staff communication mode, patient data collection mode, procedure preparation mode, patient data visualization mode, patient data analysis mode; generating experience data for adapting an experience based on the determined selection mode.

Optionally, the method may further comprise the step: generating experience data for adapting an experience based on the determined selection mode. For example, the selection mode "individual mode" may be determined. For each staff and patient experience data, i.e. control data and content information for respective devices, is generated. For example, in case content is projected on a wall in the medical environment, respective positions of the projections on the wall and respective content (e.g., procedure information for staff and an image of a mountain for the patient) generated in order to comply with different viewing angles of patient and staff.

Optionally, the method may further comprise obtaining workflow progress data; using the obtained workflow progress data for determining the selection mode and using the obtained workflow progress data for generating the experience data.

Optionally, the workflow progress data may obtained by tracking. The tracking may comprise tracking one or more device settings and or one or more usages of devices. The tracking may comprise tracking an activity of staff and/or a patient. The tracking may be carried by one or more sensors. The tracking may be carried out in real time. The tracking may comprise analyzing control data of one more devices in the medical environment. Optionally, the method may further comprise obtaining staff activity data, and using the obtained staff activity data for determining the selection mode and/or obtaining patient state data, and using the obtained patient state data for determining the selection mode; and/or obtaining staff state data, using the staff state data for determining the selection mode; and/or obtaining patient activity data, using the patient activity data for determining the selection mode. The staff activity data, the staff state data, the patient activity data, the patient state data may be obtained by using one or more sensors, analyzing usage of one or more devices, analyzing of input to user interfaces as described above.

Optionally, the method may further comprise obtaining prior data, wherein the prior data comprises one more of the following: patient monitoring data, EMR data, comparative case data with similar workflow and/or protocols including used device data, staff profile data and using the obtained prior data for determining the selection mode.

Optionally, the obtained workflow information data and/or the obtained staff activity data and/or obtained patient state data and/or obtained prior data and/or the obtained staff state data and/or the obtained patient activity data may be used for generating the experience data.

Fig. 2 shows a schematic diagram of a method according to another exemplary embodiment of the present disclosure. The diagram shows five data streams 100 to 104 transporting information into the selection mode model 105. The first data stream relates to workflow information data 100. The workflow information data may stem from a hospital data base. The second data stream relates to workflow progress data 101. The workflow progress data may obtained by tracking as described above. The third data stream relates to patient state data 102. The patient state data may obtained by tracking as described above. The fourth data stream relates to staff activity data 103. The staff activity data may be obtained by tracking. The fifth data stream relates to prior data 104. The prior data may relate in the present example to patient monitoring data from a previous procedure. The prior data may be obtained via an interface of a hospital data base. The experience mode model 105 receives the respective data streams 100 to 104 and processes the information for example by weighting them. The experience mode model 105 further selects based on the received information a selection mode 106, e.g., an individual mode. Based on the selected selection mode the method generates experience data 107 that is used for controlling one or more devices in medical environment.

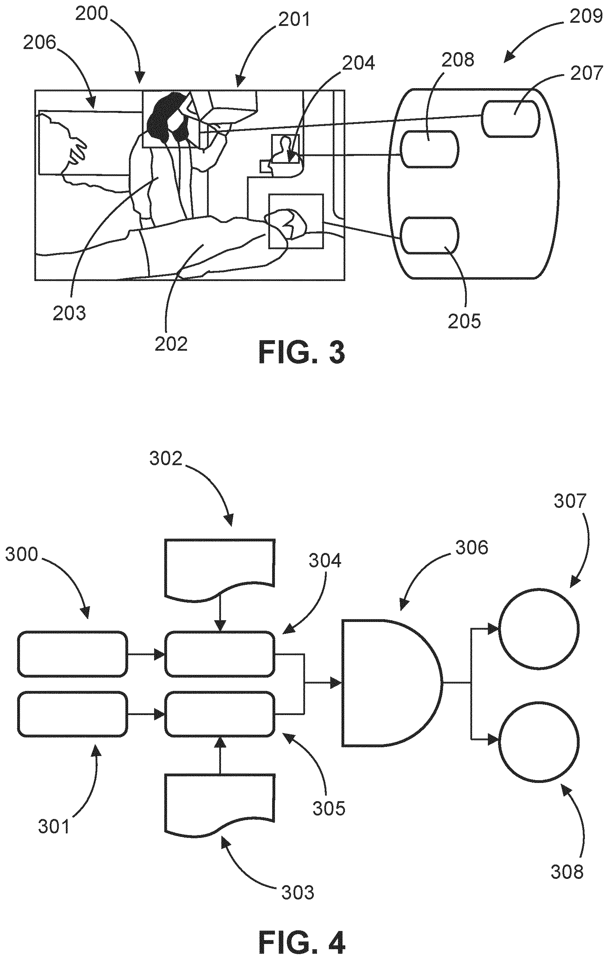

Fig. 3 shows a schematic illustration of an individual mode according to an exemplary embodiment of the present disclosure. Fig. 3 shows a medical environment 200. The medical environment 200 in the present example is an IGT system. The IGT system comprises an imaging system 201. A patient 202 is lying on a patient bed below the imaging system 201. A physician 203 prepares a therapy. And a staff member 204 is additionally present in the medical environment. In the individual mode for each of the three users a specific content is created by employing the method described above. For the patient 202 content 205 is created and shown on a wall 206 of the room in the medical environment 200. For physician 203 content 207 is created and shown on another wall or on the same wall. For the staff member 204 content 208 is created and projected on another wall or the same wall as for the physician 203 (not shown). Each content is positioned such that it matches a respective viewing angle of the patient, the physician and the staff member. The content 205, 207 and 208 is projected by a multi view display 209. As input for positioning the content head positions of the patient, the clinician and the staff member may be tracked used by the method described above. This may allow to provide different experiences for the patient, the physician and the staff member by showing respective individual content for each view.

Fig. 4 shows a schematic illustration of an individual mode according to an exemplary embodiment of the present disclosure. The figure shows how the method generates experience data based on workflow information data, patient activity data, and staff activity data. The patient activity data 300 is obtained by head tracking of the patient. The staff activity data 301 is obtained by head tracking of the staff. Workflow information data 302 is obtained by querying a database. Additionally, the method receives imaging content data 303 from a data base after determining a workflow step and selecting a selection mode, in this example individual mode. The method may further create based on the imaging content data 303, patient activity data 300, staff activity data 301, workflow information data 302 a first individual content 304 for the patient and a second individual content 305 for the staff. The method may provide the created first individual content 304 and the second individual content 305 and corresponding positioning information data to a display 306. The first individual content 304 may comprise appropriate content from a library (e.g., mountain images). The second individual content 305 may comprise clinical information (e.g., checklists, informative content, progress data,) as well as real time scanning data (e.g., imaging data of the patient). The second individual content 305 may be generated in real time. The display may be autostereoscopic display (e.g., lenticular display, parallax barrier display, holographic display). The display may be a digital light processing display. By controlling characteristics of light reflected from different mirrors and by moving the mirrors different content can be generated for different viewer positions. The display 306 may then show a first individual content 307 for the patient and a second individual content 308 for the staff.

Alternatively, the method may use patient-height information and expected positioning of the patient with respect to the imaging table (this information which can be learned from imaging protocol) to determine the space regions from which content for the patient is visible.

Alternatively, the method may use staff-height information together with the control-room positioning information, and the expected actions to be performed by the staff (determined from the imaging protocol) to determine the space regions from which staff-related information is visible.

In the following some aspects of the method are explained in combination with an IGT system. The IGT workflow may be distinguished in the following workflow steps: preparation holding in a holding area (A), arrival in lab and preparation (B), waiting for physician in lab (C), procedure performed by physician in lab (D), patient prepared to leave lab (E), patient waits and recovers in the recovery area (F). For each workflow step the following aspects can be taken into account to select a selection mode and to generate experience data.

### Step A

- The experience can be provided via special personal devise such as smart phone, or virtual reality glasses. The experience may be personalized to the patient and procedure.
- In case, the patient is tracked by a monitoring device, corresponding patient state data and patient activity data can be used beside the workflow information data and/or protocol information data to determine a selection mode and to generate experience data for adapting an experience.

### Step B

- This may be the most stressful moment for the patient. Therefore, all attention should be on the patient. To achieve this, all ambient experience resources can be targeting the patient needs. To motivate (and remind) nurses to focus on the patient a joint AE (i.e. shared mode) can be provided in the beginning.
- Only after the patient is sufficiently relaxed, or after a minimum amount of time has passed, the dual experience (i.e. individual mode) may be activated. The dual experience may help staff make the preparations more efficiently and accurately by helping them to see checklists, reminders, progress about the preparations.
- The dual experience may help the staff with the preparations by distracting the patient so that the patient's engagement with the nurses will be minimal (to help them do preparations without being disturbed).
- At some points during the preparation, typically towards the end of the preparations, the staff may need to provide specific instructions to the patient and interact with the patient. To allow for improved communication between the staff and patient, a joint AE that helps the communication can be selected. For example, an experience that makes it easy to hear speech and see faces. Speech can be automatically recognized, and summary texts can be shown on screens to help both the patient and staff to follow and build upon the conversation.

### Step C

- Both staff and patient are waiting for an undefined period of time, which can be long. In the beginning of the waiting, patient and staff can be engaged in a conversation where additional information about the procedure can be provided, or where personal information (small chat) are exchanged. The AE (i.e. staff patient communication mode of the method) can support the conversation by creating a relaxed and conversation friendly settings.
- Later, both the patient and staff may need to relax in their own. Dual experience can be used to provide different relaxing content to staff and the patient. The dual experience can start when the end of the conversation is automatically detected.

### Step D

- All the focus may be on creating the optimal settings for the procedure. The AE (here experience) may be created so that it will not distract the physician and nurses.
- Dual experience may be used, where patient experience will target distracting and entertaining the patient during the procedure, without negatively influencing the procedure (e.g., without creating glare on screens, sound, etc.)
- Experience towards the staff can include procedural information to help with performing the procedure, or preventing common clinical errors.

### Step E

- Patient is informed by staff about how the procedure went. At this stage joint experience may be used.
- After the communication, dual experience may be used to enable staff focus on their work, while patient is being entertained.

### Step F

- Most probably patient may be recovering here alone (or with family members) with some visits from the clinicians. A single AE targeting the patient and family members can be suitable in this case.
- Cath-lab staff may be busy preparing the cath-lab for the next patient. Whilst doing this ambient experience can be used to create a more work-friendly environment. Since staff may be usually working in a windowless environment, projections can be used to create the perception of windows or other soothing elements. In addition, information on the next procedure (equipment to be prepared, checklists) can be projected to aid the personnel in the preparation process.

In the following some aspects of the method are explained in combination with a use of the method in an MRI system. The MRT workflow may distinguished in the following workflow steps: waiting room (A), patient preparation in changing room (B), patient preparation in preparation room (C), patient installation in scan room (D), scanning in scan room (E), End of scan in scan room (F), End of Scan in changing room (G).

### Step A

- In the waiting room the patient can be prepared for the exam with exam specific information. Double ambient experience can be used to project patient specific information to each individual patient in the waiting room.

### Step B

- Whilst in the changing room the patient is alone and ambient experience can be focused on the patient solely. Reminders about what materials should be removed in the changing room can be provided along with soothing materials to calm the patient.

### Step C

- The staff may need to provide specific instructions to the patient, and interact with the patient. To allow for improved communication between the staff and patient, a joint AE that may help the communication can be selected. For example, an experience may be provided that projects procedure specific instructions and animations to help the patient better understand what is about to happen.
- The dual experience may help staff make the preparations more efficiently and accurately by helping them to see checklists, reminders, progress about the preparations and instructions given to the patient.

### Step D

- Entering the exam room may be the most stressful moment for the patient. Therefore, all attention should be on the patient. To achieve this, all ambient experience resources can be targeting the patient needs. To motivate (and remind) technicians to focus on the patient a single AE can be provided in the beginning.
- Later, Ambient experience can be used to provide instructions to the technician on correct positioning of the patient and correct use and placement of coils. Warnings can be given on possible bore colliding materials. Furthermore, checklists, reminders and progress about the preparation can be provided.

### Step E

- All the focus may be on creating the optimal settings for the procedure. The AE may be created so that it will not distract the technicians
- Dual experience may be used, where patient experience will target distracting, comforting and compliance to instructions like breath holding and lying still
- Experience towards the staff can include procedural information to help with performing the procedure, or preventing common clinical errors.

### Step F

- Patient may be informed by staff about how the procedure went. At this stage joint experience may be used.

### Step G

- Back in the changing room experience can be focused on the patient again. The experience may remind the patient not to forget their personal belongings and some instructions for leaving the department after finishing in the dressing room. A calming atmosphere may be created to calm the patient from all that has been experienced before and during the scan.

### LIST OF REFERENCE SIGNS

- S100: providing an experience mode model
- S200: obtaining workflow information data and/or protocol information data
- S300: generating experience data
- S400: providing generated experience data

- 100: workflow information data
- 101: workflow progress data
- 102: patient state data
- 103: staff activity data
- 104: prior data
- 105: experience mode model
- 106: selection mode
- 107: experience data
- 200: medical environment
- 201: imaging system
- 202: patient
- 203: physician
- 204: staff
- 205, 207, 208: content
- 206: wall
- 209: multi view display
- 300: patient activity data
- 301: staff activity data
- 302: workflow information data
- 303: imaging content data
- 304: first individual content
- 305: second individual content
- 306: display
- 307: display of first individual content
- 308: display of second individual content

## Claims

1. A computer implemented method for providing experience data for adapting an experience in a medical environment, comprising the steps:
providing an experience mode model configured to describe a relation between workflow information data and/or protocol information data and experience data for adapting an environment (S100);
obtaining workflow information data and/or protocol information data (S200);
generating experience data for adapting an experience based on the obtained workflow information data and/or protocol information data and the experience mode model (S300);
providing the generated experience data for further processing (S400).

2. The method according to claim 1,
wherein the experience mode model is configured to describe a relation between workflow information data and/or protocol information data and selection modes for an experience in the medical environment; and the method further comprises the steps:
determining a selection mode of a plurality of selection modes based on the workflow information data and/or the protocol information data and the experience mode model;
generating experience data for adapting an experience based on the determined selection mode.

3. The method according to claim 2, wherein the plurality of selection modes comprises a shared mode for a joint experience for a staff and a patient and/or an individual mode for individual experiences for a staff and a patient.

4. The method according to claims 2 or 3, wherein the plurality of selection modes comprises one or more of the following: patient staff communication mode, inter staff communication mode, patient data collection mode, procedure preparation mode, patient data visualization mode, patient data analysis mode.

5. The method according to any one of the preceding claims, further comprising:
obtaining workflow progress data;
using the obtained workflow progress data for determining the selection mode; and/or
using the obtained workflow progress data for generating the experience data.

6. The method according to claim 5, wherein obtaining workflow progress data comprises a tracking.

7. The method according to any one of the preceding claims, further comprising:
obtaining staff activity data, and
using the obtained staff activity data for determining the selection mode and/or
obtaining patient state data, and
using the obtained patient state data for determining the selection mode; and/or
obtaining staff state data,
using the staff state data for determining the selection mode; and/or
obtaining patient activity data,
using the patient activity data for determining the selection mode.

8. The method according to any one of the preceding claims, further comprising:
obtaining prior data, wherein the prior data comprises one more of the following: patient monitoring data, EMR data, comparative case data with similar workflow and/or protocols including used device data, staff profile data;
using the obtained prior data for determining the selection mode.

9. The method according to any one of the preceding claims, wherein the obtained workflow information data and/or the obtained staff activity data and/or the obtained patient state data and/or the obtained prior data and/or the obtained staff state data and/or the obtained patient activity data is used for generating the experience data.

10. The method according to any one of the preceding claims, wherein the further processing comprises
controlling one or more devices in the medical environment based on the experience data for generating a desired environment, wherein controlling comprises one or more of the following:
positioning of the device, adapting the device, selecting and transmitting of content, adapting a user interface.

11. The method according to claim 10,
wherein the controlling is carried out differently for two or more patients and/or staff in the medical environment.

12. The method according to any one of the preceding claims, wherein the medical environment is one of the following: a CT system, an IGT system, a DXR system, a MRI system.

13. The method according to any one of the preceding claims, wherein the selection mode model is based on one or more of the following: a look up table, a weight function, a machine learning algorithm.

14. A data processing apparatus comprising means for carrying out the method of any one of the claims 1 to 13.

15. A computer program comprising instructions which, when the program is executed by a computer, cause the computer to carry out the method according to any one the claims 1 to 13.

16. A computer-readable medium comprising instructions which, when executed by a computer, cause the computer to carry out the method according to any one of the claims 1 to 13.
